# EUROPEAN PATENT APPLICATION

(11) **EP 4 431 117 A1**
(43) Date of publication of application: **18.09.2024**
(21) Application number: 22893192.9
(22) Date of filing: 09.11.2022
(51) Int. Cl.: A61K 48/00, A61K 31/7105, A61P 9/00, C12Q 1/6883

(54) **COMPOSITION FOR DIAGNOSIS, PREVENTION, OR TREATMENT OF VASCULAR SMOOTH MUSCLE CELL PROLIFERATIVE DISEASE, USING MIRNA INHIBITOR**

(30) Priority: 09.11.2021 KR 20210153320
(71) Applicant: Ewha University-Industry Collaboration Foundation, Seoul 03760 (KR); VASTHERA Co. Ltd., Seoul 03760 (KR)
(72) Inventor: KANG, Sang Won, Seoul 06517 (KR); LEE, Sang-Hyuk, Seoul 04111 (KR); KIM, Yerin, Seoul 07213 (KR)
(74) Representative: Ström & Gulliksson AB
(86) International application number: PCT/KR2022/017553
(87) International publication number: WO 2023/085772

(57) **Abstract**

The present invention relates to a pharmaceutical composition comprising a microRNA (miRNA) inhibitor as an active ingredient for the prevention or treatment of a vascular smooth muscle cell proliferative disease; a kit comprising a miRNA-detectable agent for the diagnosis of a vascular smooth muscle cell proliferative disease; a method for providing information for the diagnosis of the diseases; and a therapeutic method. Discovered in the present invention were the miRNA subsets miR-132-3p, miR-370-3p, miR-130b-5p, and miR-410-3p that are involved in proliferative diseases of smooth muscle cells. It was found that these miRNAs significantly increased their expression 5-fold or more in damaged arteries, compared to the control, and in particular, miR-370-3p was highly expressed in coronary tissues of atherosclerosis patients. Thus, the miRNAs can be advantageously used for diagnosing diseases related thereto and further can find applications in treating vascular smooth muscle cell proliferative diseases.

## Description

### [Technical Field]

The present invention relates to a pharmaceutical composition containing a microRNA (miRNA) inhibitor as an active ingredient for the prevention or treatment of a vascular smooth muscle cell proliferative disease, to a kit including an agent capable of detecting a miRNA for the diagnosis of a vascular smooth muscle cell proliferative disease, to an information-providing method for diagnosing the disease, and to a treatment method.

### [Background Art]

Normal arterial vessels are composed of two major cell types: heathy endothelial cell (EC) monolayers and contractile smooth muscle cells (smooth muscle cells). However, the intimal accumulation of low-density lipoproteins combined with disturbed blood flows may inflame ECs, and monocytes/macrophages attach onto the inflamed EC lesion to penetrate the EC monolayer and take up lipid particles, resulting in foam cells. The resultant macrophage-driven foam cells and immune cells in the atherosclerotic lesion secrete many growth factors and cytokines that stimulate smooth muscle cells for dedifferentiation and fibrous cap formation. The entire atherogenesis process, including initial inflammatory responses, eventually causes the occlusion of arterial lumens. Currently, angioplasty along with stent procedures is the only method known to repair the occluded arteries in patients with atherosclerosis. However, the major complication post-procedure is thrombosis resulting from endothelial damage, which causes additional neointimal hyperplasia, called in-stent restenosis. This restenosis complication procedure resembles the late stage of atherogenesis in terms of the proliferation and migration of dedifferentiated smooth muscle cells. This indicates that no targeted approach can be the right solution to prevent atherogenesis.

Since microRNA (abbreviated as miRNA) was discovered during the study of the lin-4 gene in nematodes in 1993, the function of miRNA as a translational regulator has received a lot of attention in the past decades. The unique feature of miRNA is to simultaneously regulate the expression of many target genes at the 3' UTR. Indeed, attempts have made to seek and characterize the vascular miRNAs that regulate the growth of smooth muscle cells (Farina, 2020 #2619; Torella, 2018 #2618; and Ji, 2007 #2617). Nevertheless, there was no genome-wide screening of vascular miRNAs common in rodents and humans.

### [Disclosure]

### [Technical Problem]

Under this situation, the present inventors have made intensive research efforts to screen vascular miRNAs associated with the over-proliferation and migration of arterial smooth muscle cells. As a result, the present inventors have identified four important miRNAs that regulate the over-proliferation and migration of arterial smooth muscle cells by using a standard animal model. In particular, they revealed that miR-370-3p is a novel miRNA that is significantly relevant to proliferative diseases of smooth muscle cells in atherosclerosis, thus completing the present invention.

### [Technical Solution]

An aspect of the present invention is to provide a pharmaceutical composition containing a microRNA (miRNA) inhibitor as an active ingredient for the prevention or treatment of a smooth muscle cell proliferative disease.

Another aspect of the present invention is to provide a kit including an agent capable of detecting a miRNA for the diagnosis of a vascular smooth muscle cell proliferative disease.

Still another aspect of the present invention is to provide an information-providing method for diagnosing a vascular smooth muscle cell proliferative disease.

Still another aspect of the present invention is to provide a method for treating a vascular smooth muscle cell proliferative disease, the method including administering to a non-human subject in need thereof the pharmaceutical composition containing a miRNA expression inhibitor.

### [Advantageous Effects]

The present invention has discovered that a subset of miRNAs (miR-132-3p, miR-370-3p, miR-130b-5p, and miR-410-3p) associated with vascular smooth muscle cell proliferative diseases, wherein these miRNAs showed a notable increase in expression by at least 5-fold in injured arteries compared with controls, and particularly, miR-370-3p showed high expression in the coronary tissue in patients with atherosclerosis, and therefore, these miRNAs can be advantageously used in the diagnosis of the disease and can further be utilized for preventing or treating vascular smooth muscle cell proliferative diseases.

### [Brief Description of Drawings]

FIG. 1A shows the heatmap analysis on differentially expressed miRNAs at days 3 and 5 after artery injury compared with sham control from the balloon injured carotid artery animal models.
FIG. 1B shows 8 up-regulated miRNAs and 2 down-regulated miRNAs showing at least 5-fold change in expression level among the differentially expressed miRNAs.
FIG. 1C shows a graph confirming that six out of the eight up-regulated miRNAs were prominently expressed in the human smooth muscle cells.
FIG. 2A shows graphs confirming that miR-132-3p and miR-370-3p exhibited significant inhibitory effects on smooth muscle cell proliferation and miR-130b-5p, miR-132-3p, and miR-410-3p significantly inhibited monocyte adhesion, as a result of examining the roles of the up-regulated miRNAs by using human aortic smooth muscle cells transfected with miRNA inhibitors.
FIGS. 2B and 2C show graphs confirming that miR-132-3p and miR-370-3p inhibitors induced cell cycle arrest at the G1 phase, thereby suppressing the time-dependent proliferation of human aortic smooth muscle cells.
FIG. 2D shows graphs confirming that miR-132-3p and miR-370-3p inhibitors markedly augmented the levels of the cyclin-dependent kinase inhibitors p21 and p27.
FIG. 2E shows graphs confirming that miR-132-3p was involved in the phenotype transition of smooth muscle cells through the conversion of smooth muscle cells into the contractile phenotype by the restoration of the SMA level with the miR-132-3p inhibitor.
FIG. 2F shows images re-confirming through F-actin filament that the synthetic phenotype of human aortic smooth muscle cells was transformed into the contractile phenotype by the miR-132-3p inhibitor.
FIG. 3A confirms that miR-132-3p and miR-370-3p were markedly increased in the balloon-injured carotid arteries and that miR-130b-5p and miR-410-3p were induced by the balloon injury, as a result of examining the arterial expression levels of four selected miRNAs through in situ hybridization.
FIG. 3B shows images and a graph confirming that the catheter-mediated local intramural delivery of four selected miRNA inhibitors markedly reduced the neointimal hyperplasia in the balloon-injured lesion compared with the control.
FIG. 3C shows the anti-proliferation efficacy in EC because the miR-130b-5p inhibitor among the miRNAs facilitated the recovery of the EC monolayer through re-endothelialization.
FIG. 4A shows the profiling of differentially expressed genes (DEGs) in the injured arteries.
FIG. 4B shows up-regulated and down-regulated DEGs through heatmap analysis.
FIG. 4C shows graphs confirming through real-time PCR that predicted target genes of four selected miRNAs were down-regulated in the balloon-injured carotid arteries compared with the sham control.
FIG. 4D shows graphs confirming through real-time PCR that the target genes of miR-132-3p, miR-370-3p, miR-130b-5p, and miR-410-3p miRNA are SOCS2, BMP7, TSPAN2, and SMAD6, respectively.
FIG. 5A confirms a higher expression of miR-370-3p in the coronary tissue sections from human patients with atherosclerosis type II and IV lesions.
FIG. 5B shows that serum stimulation induced miR-370-3p expression but reduced BMP7 expression, as a result of examining the relationship between miR-370-3p and BMP7.
FIG. 5C confirms that inhibition of miR-370-3p increased the protein level of BMP7 in vascular smooth muscle cells.
FIG. 5D confirms that the miR-370-3p mimic reduced the expression of luciferase containing the wild-type UTR but not the negative control, in which two consecutive nucleotides in the miR-370-3p target region of the human BMP7-3' UTR were mutated.
FIG. 5E confirms that the BMP7 treatment induced the SMAD1/5/9 phosphorylation in vascular smooth muscle cells.
FIG. 5F demonstrates that the reduction of BMP7 by siRNA transfection recovered the proliferation of smooth muscle cells, which had been inhibited by the miR-370-3p inhibitor.

### [Detailed Description of the Invention]

The present invention will be specifically described as follows. However, the scope of the present invention is not limited by the specific description below. Each description and embodiment disclosed herein may also be applied to other descriptions and embodiments, and all combinations of various elements disclosed in the present invention fall within the scope of the present invention.

In accordance with an aspect of the present invention, there is provided a pharmaceutical composition for the prevention or treatment of a vascular smooth muscle cell proliferative disease, the pharmaceutical composition containing a microRNA (miRNA) inhibitor as an active ingredient.

The present invention also provides the use of the microRNA (miRNA) inhibitor for the prevention or treatment of a vascular smooth muscle cell proliferative disease.

As used herein, the term "miRNA", which may be used interchangeably with microRNA or the like, refers to a small RNA that serves to regulate gene expression in organisms and, specifically, is a small RNA of 20 to 25 nucleotides that can play a key role in gene expression through the inhibition of target mRNA translation by complementary base pairing with the 3' untranslated region (UTR) of the target mRNA. The miRNA plays an important role in cellular functions including proliferation, differentiation, and apoptosis, and is an evolutionarily conserved regulator present in all animals. Some miRNAs may regulate gene expression through epigenetic regulatory mechanisms (histone modification, DNA methylation, etc.) associated with promoter sites thereof.

The miRNAs used in the present invention regulate over-proliferation, phenotypic transition, migration, and the like of smooth muscle cells, and specifically, miR-132-3p, miR-370-3p, miR-130b-5p, and miR-410-3p were determined through a balloon injury artery model. In addition, the key target genes thereof were further identified, and especially, miR-370-3p was discovered to be a novel miRNA associated with atherosclerosis and was first established by the present inventors.

As used herein, the term "miRNA inhibitor" refers to an agent that reduces the expression or activity of each miRNA in a cell, and the term may mean blocking miRNA that regulates the target gene expression by acting directly on each miRNA or acting indirectly on an upstream regulator. In the present invention, the miRNA inhibitor may mean regulating vascular smooth muscle cell functions and in vivo neointimal hyperplasia by inhibiting the up-regulated expressed miRNA in the injured arteries, and specifically, may indicate each inhibitor of miR-132-3p, miR-370-3p, miR-130b-5p, or miR-410-3p, but is not limited thereto.

In the present invention, the miRNA inhibition may be achieved by using an inhibitor specific to miR-132-3p, miR-370-3p, miR-130b-5p or miR-410-3p, and specifically, examples of the inhibitor may be an siRNA, aptamer, antisense oligonucleotide, ribozyme, or compound, specific to each miRNA, but is not limited thereto. For the purposes of the present invention, the compound may employ any compound that inhibits miRNAs associated with in vivo neointimal hyperplasia.

The injured arteries result from artificial injury of arteries in order to screen vascular miRNAs associated with the over-proliferation of arterial smooth muscle cells, for the purposes of the present invention, and specifically, a model was created by balloon-induced injury of the rat carotid artery and used in experiments.

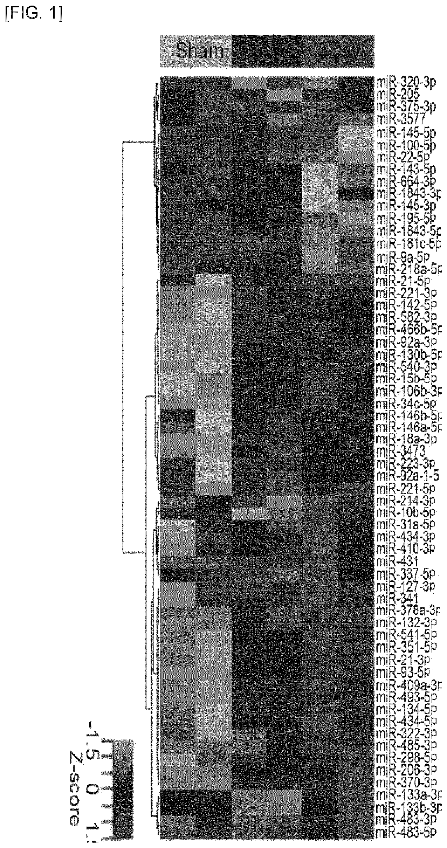

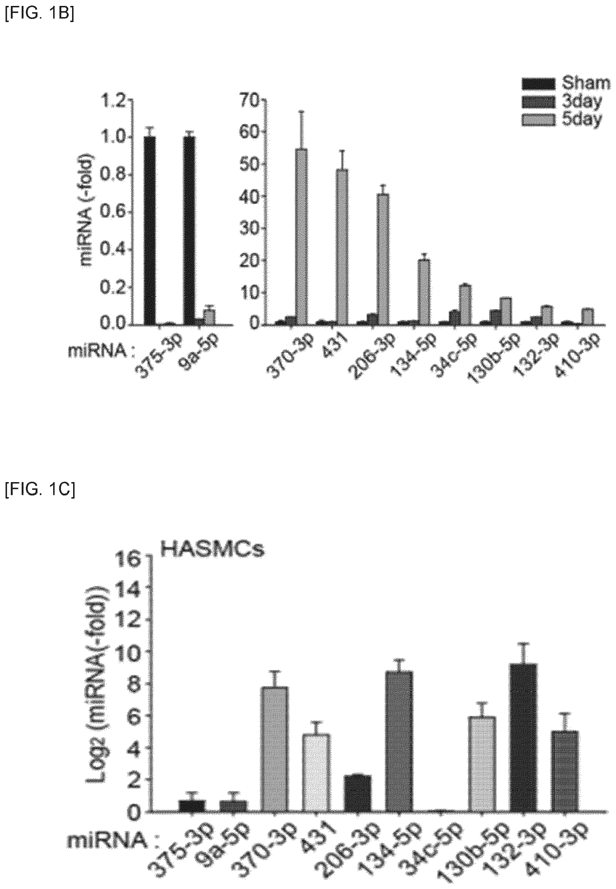

In one example of the present invention, differentially expressed miRNAs in the injured arteries were examined, and as a result, among 62 differentially expressed miRNAs at days 3 and 5 after artery injury compared with the control, 6 up-regulated miRNAs were finally identified by examining the expression thereof in the human aortic smooth muscle cells (FIG. 1).

The term "upregulated" refers to the increase in mature miRNA with increasing transcription and processing of a specific miRNA, and in the present invention, the term may mean the increase in biosynthesis in an artery injury model compared with a control group. For the purposes of the present invention, the increase in expression may specifically indicate a notable increase by at least 5-fold compared with the control group, but is not limited thereto.

As used herein, the term "vascular smooth muscle cells" refers to cells that constitute the inner wall of blood vessels and function to maintain the blood pressure constant through contraction and relaxation. In normal vessels, smooth muscle cells differentiate to produce proteins necessary for contraction and relaxation, after which cell proliferation ceases. However, smooth muscle cells dedifferentiate due to cell dysfunction, resulting in narrowed vessels, causing diseases, such as atherosclerosis and vascular restenosis. These vascular diseases are closely associated with functions of vascular smooth muscle cells, and therefore, a vascular smooth muscle cell model is an important cellular model in vascular disease research.

As used herein, the term "proliferation" refers to increasing the number of organisms or tissue cells through cell division and usually means increasing cells in the body of multicellular organisms. In the present invention, the cell proliferation may mean the proliferation of vascular smooth muscle cells, and the over-proliferation of vascular smooth muscle cells is an important factor in the progression of atherosclerosis lesions.

As used herein, the term "vascular smooth muscle cell proliferative disease" refers to a disease caused by over-proliferation of vascular smooth muscle cells. The vascular smooth muscle cell proliferative disease may include not only vascular stenosis, vascular restenosis, atherosclerosis, and atherosclerotic arteriosclerosis, which are directly caused by the proliferation of vascular smooth muscle cells, but also cardiovascular diseases, which are secondarily caused or have worsened symptoms by those disease, for example, heart failure, myocardial infarction, angina, arrhythmia, hypertensive heart disease, congenital heart disease, stroke, and peripheral vascular stenosis.

In the present invention, the vascular smooth muscle cell proliferative disease may be specifically vascular stenosis, vascular restenosis, atherosclerosis, and atherosclerotic arteriosclerosis.

The vascular stenosis is a disease in which the inside of the vessel is abnormally narrowed and the blood flow rate is reduced due to inflammation, blood clots, and over-proliferation of smooth muscle cells after the injury of the vascular wall. The vascular restenosis indicates the reoccurrence of vascular stenosis. In most cases, the vascular restenosis occurs after vascular procedures, such as widening the vascular lumen or unclogging blocked blood vessels to solve vascular stenosis. Vascular procedures, for example, angioplasty, such as stent/balloon angioplasty, and vascular bypass or vascular graft, such as coronary artery bypass surgery, themselves may injure the vessels or provoke inflammation, resulting in vascular stenosis. Atherosclerosis is a disease in which fat is deposited or fibrosed in the inner layer of the artery, and the progression of atherosclerosis and vascular restenosis that occurs after stent insertion to widen blood vessels are known to be all caused by the proliferation, migration, and extracellular matrix secretion of vascular smooth muscle cells.

As used herein, the term "prevention" refers to any action that inhibits or delays the outbreak of a vascular smooth muscle cell proliferative disease by administration of the pharmaceutical composition of the present invention, and the term "treatment" refers to any action that alleviates or beneficially changes the symptoms of a subject suspected of having a vascular smooth muscle cell proliferative disease by administration of the pharmaceutical composition.

The pharmaceutical composition of the present invention can inhibit the activity and/or expression of miR-132-3p, miR-370-3p, miR-130b-5p, or miR-410-3p to prevent or treat diseases associated with these miRNAs.

The pharmaceutical composition according to the present invention may contain a miR-132-3p, miR-370-3p, miR-130b-5p, or miR-410-3p inhibitor in a content of 0.1 to 75 wt%, more preferably 1 to 50 wt%, relative to the total weight of the composition.

In addition, the pharmaceutical composition may further contain complex forms with various nucleic acid carriers (viral or non-viral carriers) known in the art to increase the in vivo delivery efficiency of the miRNA inhibitors. The viral carriers, specifically, lentiviruses, adenoviruses, and adeno-associated viruses may be used to allow vectors encoding miRNA to be delivered into the nuclei of the cells to express miRNA inhibitors. Examples of the non-viral carriers may include inorganic nanoparticle-based delivery carriers, whose sizes and shapes can be adjusted using inorganic materials, such as gold, carbon, and silica, polymer-based carriers, such as PLGA and PEI, which are specially modified for effective delivery to specific cells, and lipid transporters using liposomes, which are composed of lipid bilayers and water as an inner phase and thus can encapsulate and deliver nucleic acids and the like, and these non-viral carriers can protect the miRNA inhibitors to improve stability during blood circulation.

The pharmaceutical composition may be administered as an individual medicine or in combination with other medicines and may be administered sequentially or simultaneously with conventional medicines. In addition, single or multiple dosing of the pharmaceutical composition may be performed. It is important to administer an amount capable of obtaining a maximum effect with a minimal amount without side effects in consideration of all of the factors, and the amount may be easily determined by those skilled in the art.

The pharmaceutical composition may be administered parenterally (e.g., intravenous, subcutaneous, intraperitoneal, or topical application) according to the desired method, and the dose of the pharmaceutical composition varies depending on the patient's condition and weight, the severity of the disease, the form of the drug, and the route and time of administration, but may be appropriately selected by a person skilled in the art.

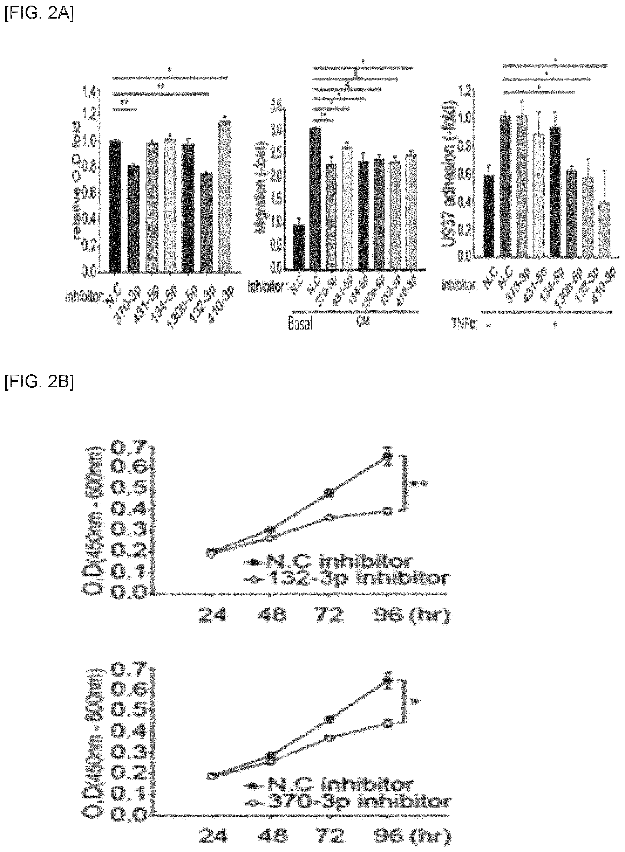

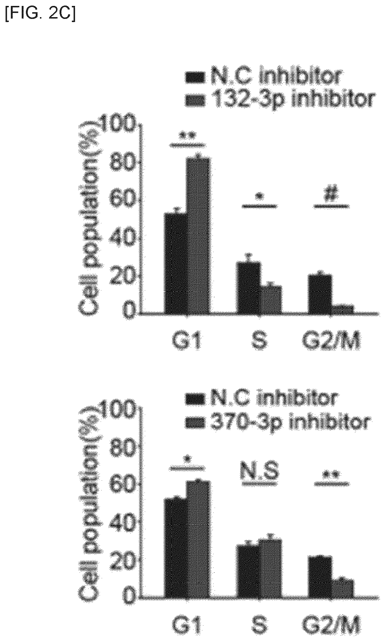

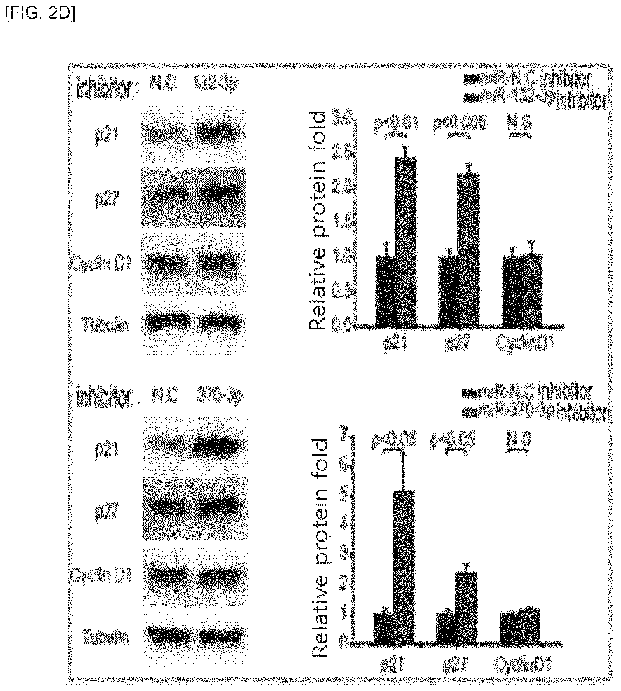

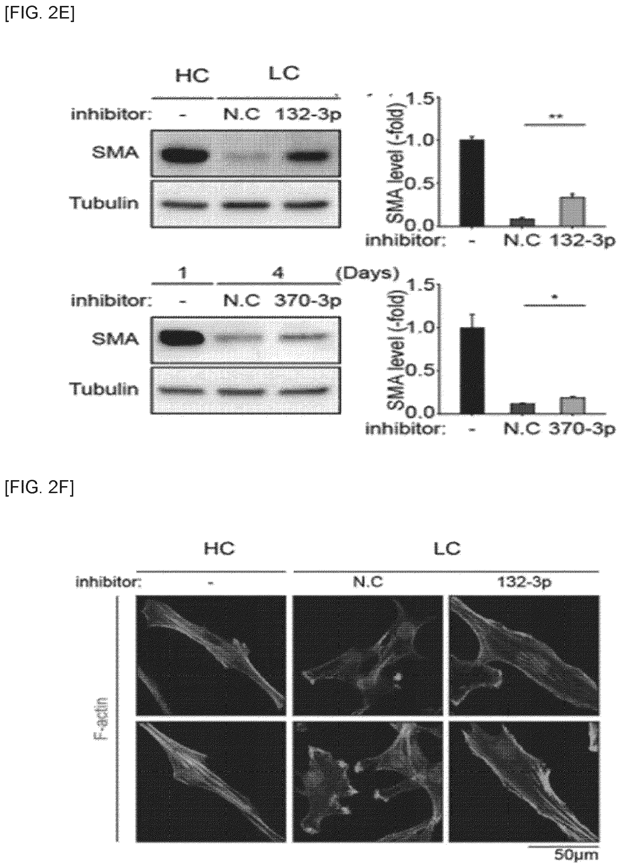

In one example of the present invention, as a result of examining up-regulated miRNAs regulating in vitro smooth muscle cell functions, miR-132-3p and miR-370-3p exhibited significant inhibitory effects on the proliferation of vascular smooth muscle cells and miR-130b-5p, miR-132-3p, and miR-410-3p significantly inhibited the monocyte adhesion to vascular smooth muscle cells. Additionally, through deep investigation into proliferation-related miR-132-3p and miR-370-3p, each is likely to regulate phenotype transition and proliferation signaling (FIG. 2).

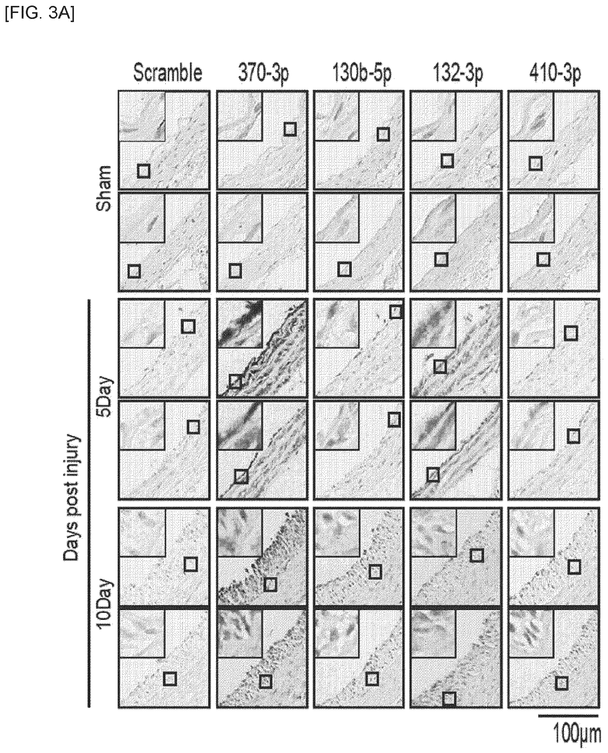

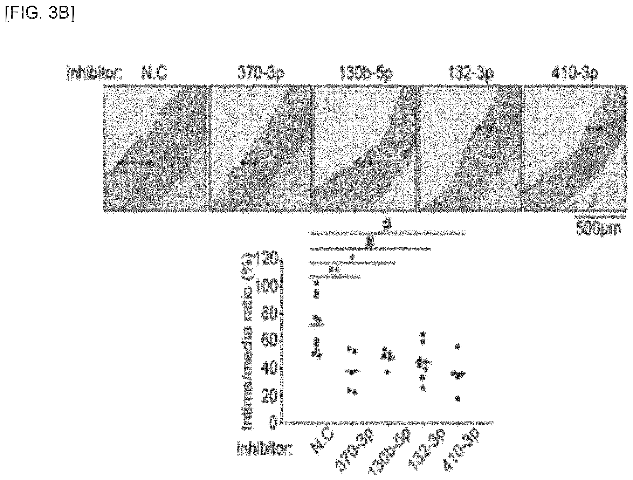

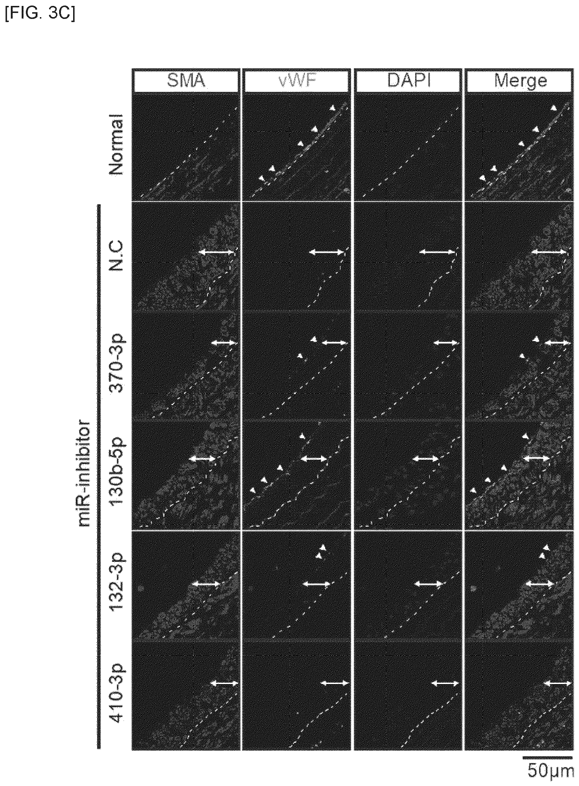

In another example of the present invention, as a result of examining the arterial expression levels of miR-132-3p, miR-370-3p, miR-130b-5p, and miR-410-3p to identify up-regulated miRNAs regulating in vivo neointimal hyperplasia, the expression of miR-132-3p and miR-370-3p was markedly increased in the balloon-injured carotid arteries compared with those in the control and miR-130b-5p and miR-410-3p were also substantially induced by the balloon injury. The miRNA inhibitors markedly reduced the neointimal hyperplasia in the balloon-injured lesion compared with the control, and the miR-130b-5p inhibitor facilitated the recovery of the monolayer, called re-endothelialization, confirming that these four miRNAs play distinct roles, such as vascular inflammation and proliferation, in arterial homeostasis (FIG. 3).

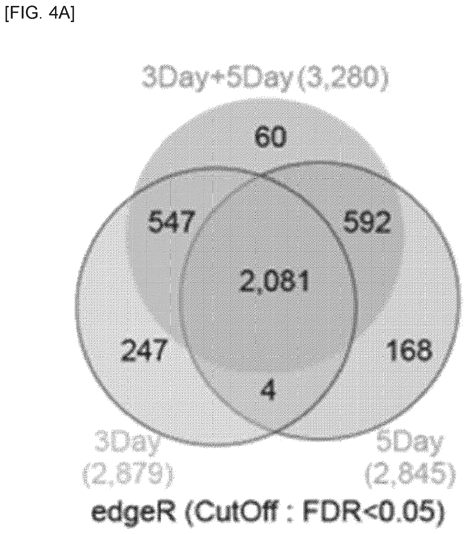

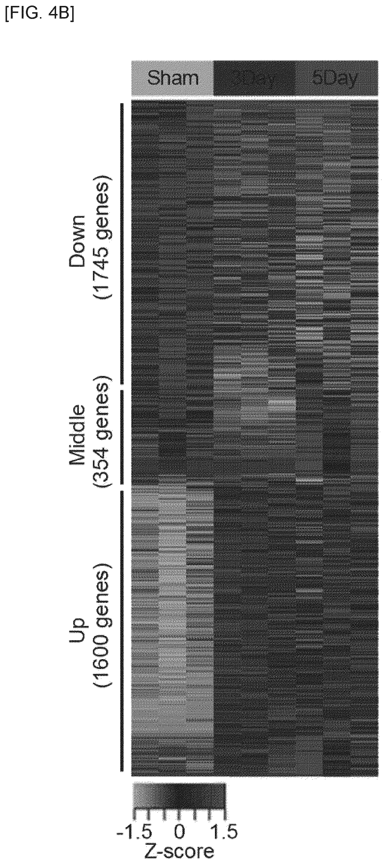

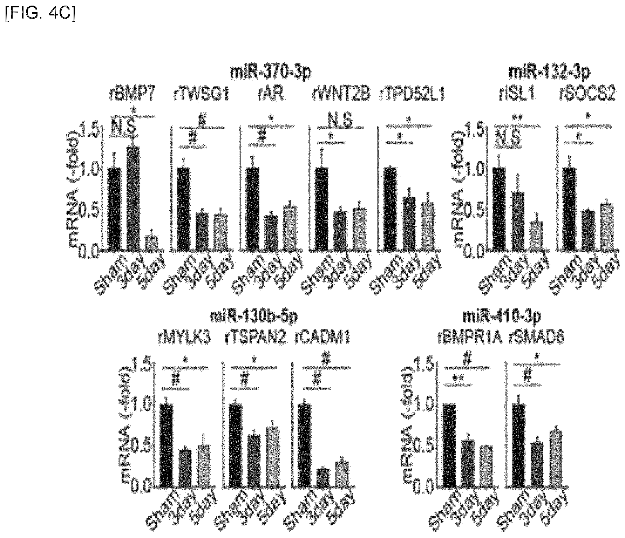

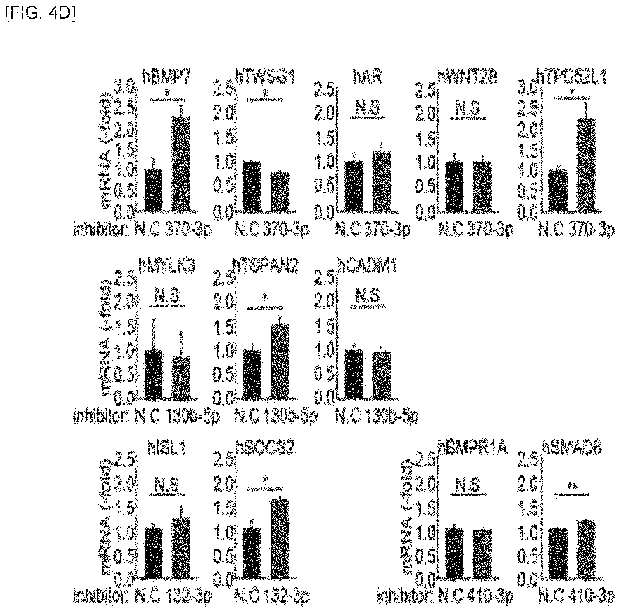

In another example of the present invention, in order to investigate key target genes for miR-132-3p, miR-370-3p, miR-130b-5p, and miR-410-3p, the differentially expressed genes (DEGs) that were commonly predicted in both rat and human through the target prediction databases were selected as target genes for each miRNA by using differentially expressed genes (DEGs) at two time points in the injured arteries, and these target genes were identified to be down-regulated in the injured arteries, revealing that the key target genes of miR-132-3p, miR-370-3p, miR-130b-5p, and miR-410-3p are suppressor of cytokine signaling 2 (SOCS2), bone morphogenetic protein 7 (BMP7), tetraspanin-2 (TSPAN2), and SMAD family member 6 (SMAD6), respectively (FIG. 4).

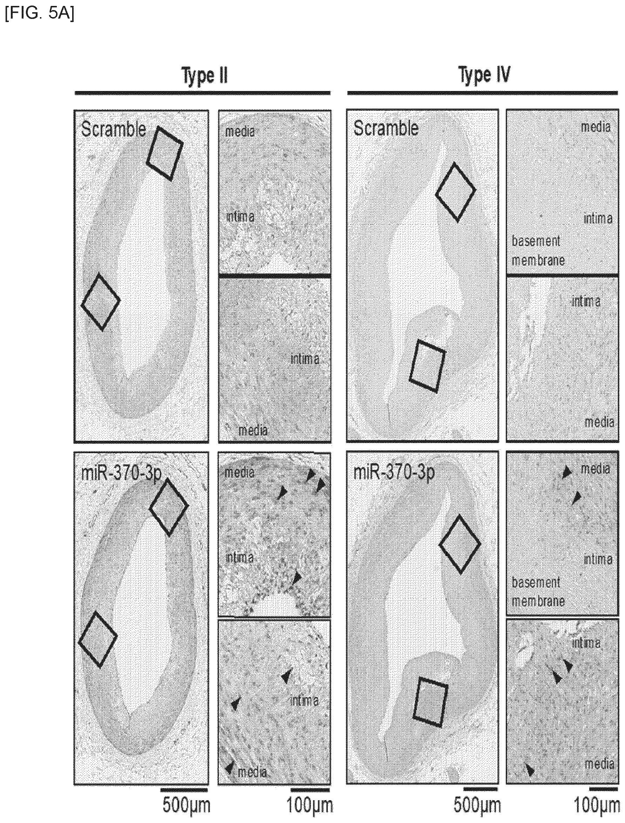

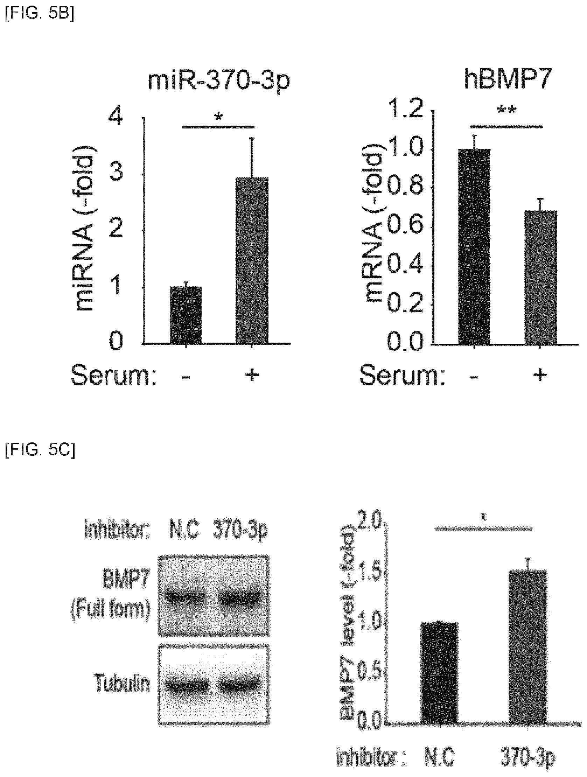

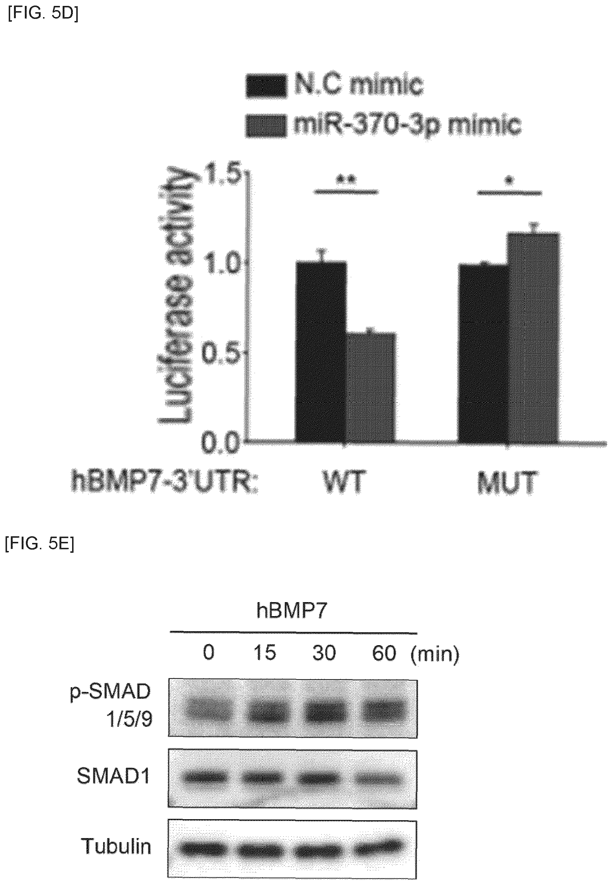

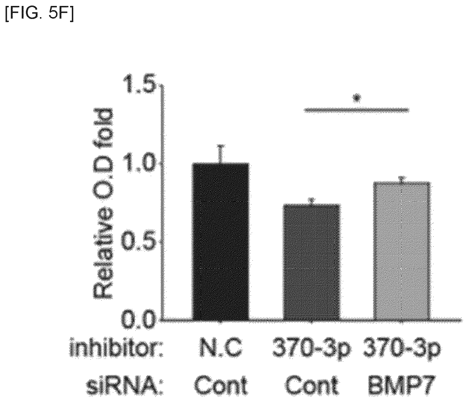

In another example of the present invention, miR-370 was detected in the blood samples of patients with unstable angina, type II diabetes, and hyperlipidemia, and thus the biological significance of the miR-370/bone morphogenetic protein (BMP)-7 axis in the proliferation of smooth muscle cells was examined, and as a result, a higher expression of miR-370-3p was identified in the coronary tissue sections from human patients with atherosclerosis type II and IV lesions. Therefore, miR-370 was a novel miRNA associated with atherosclerosis; the miR-370 inhibition increased the protein level of BMP7 in human aortic smooth muscle cells; BMP7 treatment markedly induced the SMAD1/5/9 phosphorylation in human aortic smooth muscle cells; and BMP7 depletion disturbed the inhibition of smooth muscle cell proliferation by the miR-370 inhibitor, so that it was identified that miR-370-dependent control of BMP7 expression is a prerequisite for smooth muscle cell growth and neointimal hyperplasia occurring in the injured arteries (FIG. 5).

Consequently, based on the cell proliferation inhibitory effects of miR-132-3p, miR-370-3p, miR-130b-5p, and miR-410-3p of the present invention, it can be expected that the inclusion of the miRNA inhibitors in smooth muscle cells can be used to prevent or treat a vascular smooth muscle cell proliferative disease.

In accordance with another aspect of the present invention, there is provided a kit for diagnosis of a vascular smooth muscle cell proliferative disease, the kit including an agent capable of detecting a miRNA.

The "miRNA" and "vascular smooth muscle cell proliferative disease" are as described above.

As used herein, the term "diagnosis" refers to the confirmation of the presence or characteristics of a pathological condition. For the purposes of the present invention, the diagnosis is directed to a vascular smooth muscle cell proliferative disease, and the diagnosis may be construed to mean the act of objectively determining whether there has been over-proliferation or migration of vascular smooth muscle cells in a patient of interest.

The diagnostic kit of the present invention may be used to qualitatively and/or quantitatively detect miR-132-3p, miR-370-3p, miR-130b-5p, and miR-410-3p, or the target genes of these miRNAs in a specimen.

Specifically, the kit may be selected from the group consisting of a microarray, an aptamer chip kit, an enzyme linked immunosorbent assay (ELISA) kit, a blotting kit, an immunoprecipitation kit, an immunofluorescence test kit, a protein chip kit, an RT-PCR kit, and a combination thereof and, more specifically, may be an RT-PCR kit, but is not limited thereto as long as the expression level of miRNA or a target gene thereof can be measured, but is not limited thereto.

In accordance with still another aspect of the present invention, there is provided an information-providing method for diagnosing a vascular smooth muscle cell proliferative disease, the method including: (a) measuring the expression level of a miRNA in an isolated biological sample; and (b) determining that there is a risk of developing a vascular smooth muscle cell proliferative disease when the expression level of the miRNA is increased compared with that in a control group.

The "vascular smooth muscle cell proliferative disease" and "diagnosis" are as described above.

The step of measuring the expression level of a miRNA in an isolated biological sample may mean measuring the expression level of, specifically, miR-132-3p, miR-370-3p, miR-130b-5p, or miR-410-3p, and more specifically, miR-370-3p.

The information-providing method may further include a step of determining whether the expression level of a target gene of the miRNA in the isolated biological sample is reduced compared with that in the control group, and specifically, the target gene may mean BMP7.

In addition, the information-providing method may include an agent for measuring the expression level of the miRNA or a target gene thereof. The agent for measuring the expression level may mean an agent capable of specifically binding to the miRNA or a target gene thereof to recognize or amplify the same. A specific example thereof may be an antibody, primer, or probe that specifically binds to the miRNA or a target gene thereof, but is not limited thereto, and a person skilled in the art could select an appropriate agent according to the purposes of the invention.

The agent may be directly or indirectly labeled to measure the expression level of the miRNA or a target gene thereof. Specifically, a ligand, a bead, a radionuclide, an enzyme, a substrate, a cofactor, an inhibitor, a fluorescer, a chemiluminescent material, a magnetic particle, a hapten, a dye, and the like may be used for the labeling, but are not limited thereto. As specific examples, the ligand includes biotin, avidin, streptavidin, and the like; the enzyme includes luciferase, peroxidase, beta galactosidase, and the like; and the fluorescer includes fluorescein, coumarin, rhodamine, phycoerythrin, sulforhodamine acid chloride (Texas red), and the like, but the labels are not limited thereto. Most known labels may be used as such detectable labels, and a person skilled in the art could select an appropriate label according to the purposes of the invention.

As used herein, the term "primer" refers to a nucleotide sequence having a short free 3'-hydroxyl group, which can form base pairs with the complementary template and function as a starting point for replicating the template strand. In the present invention, a primer that is used for amplification of the miRNA may be a single-stranded oligonucleotide that can act as a starting point for template-directed DNA synthesis in an appropriate buffer under appropriate conditions (e.g., four different nucleoside triphosphates and polymerizing agents such as DNA or RNA polymerase or reverse transcriptase) and appropriate temperatures, and the proper length of the primer may vary depending on the purpose of use. The primer sequence need not be completely complementary to the polynucleotide of the miRNA of the gene or its complementary polynucleotide, and any primer sequence can be used as long as it is sufficiently complementary for hybridization.

The term "probe" refers to a labeled nucleic acid fragment or peptide capable of specifically binding to a miRNA. As a specific example, the probe may be constructed in the form of an oligonucleotide probe, a single stranded DNA probe, a double stranded DNA probe, an RNA probe, an oligonucleotide peptide probe, or a polypeptide probe.

In the present invention, the isolated biological sample may specifically mean a blood or coronary tissue section from a patient with atherosclerosis type II and type IV lesions, and more specifically, the coronary tissue may mean a tissue scraped from a blocked arterial blood vessel, but is not limited thereto.

miR-370-3p has been detected in the blood of patients with unstable angina, type 2 diabetes, and hyperlipidemia, and was identified to be highly expressed in coronary tissue sections of patients with atherosclerosis, indicating that miR-370-3p is a novel miRNA associated with atherosclerosis.

The method for providing the information necessary for determining the outbreak of a disease may include quantitatively analyzing the expression level of a miRNA or a target gene thereof from the blood or coronary tissue of a subject suspected of having a vascular smooth muscle cell proliferative disease, specifically atherosclerosis.

The term "subject" may encompass, without limitation, mammals including, for example, rats, livestock, humans, and the like that are at risk of developing or have developed a vascular smooth muscle cell proliferative disease.

In accordance with still another aspect of the present invention, the present invention, for achieving the above-described purposes, may provide the use of an agent for measuring the expression level of a miRNA or a miRNA target gene in the preparation of an agent for diagnosis of a vascular smooth muscle cell proliferative disease.

The "vascular smooth muscle cell proliferative disease", "diagnosis", and "miRNA" are as described above.

In accordance with still another aspect of the present invention, there is provided a method for treating a vascular smooth muscle cell proliferative disease, the method including administering, to a subject in need thereof, a pharmaceutical composition for the prevention or treatment of a vascular smooth muscle cell proliferative disease, the pharmaceutical composition containing an miRNA expression inhibitor and a pharmaceutically acceptable carrier.

The "vascular smooth muscle cell proliferative disease", "prevention", "treatment", and "miRNA" are as described above.

In the present invention, the expression inhibitor may mean a small interfering RNA (siRNA), an aptamer, or an antisense RNA that complementarily binds to each miRNA, but is not limited thereto.

As used herein, the term "subject" may refer to any animal, including humans that has or develops the vascular smooth muscle cell proliferative disease in the present invention. The administration of the pharmaceutical composition of the present invention into a subject can lead to effects of preventing and treating the disease.

The pharmaceutical composition of the present invention is administered at a pharmaceutically effective amount.

As used herein, the term "administration" refers to the introduction of the pharmaceutical composition of the present invention into a subject by any suitable method, and the pharmaceutical composition may be administered through various parenteral routes as long as the pharmaceutical composition can reach a target tissue.

The pharmaceutical composition may be administered to a subject as appropriate according to a common method, route of administration, and dose, which are used in the art as intended or needed. Examples of the route of administration include parenteral, subcutaneous, intraperitoneal, intrapulmonary, and intranasal administrations, and examples of the parenteral injection include intramuscular, intravenous, intraarterial, intraperitoneal or subcutaneous administration. Additionally, the dose and the number of administration may be appropriately selected according to a method known in the art, and the dose and the number of administration of the pharmaceutical composition of the present invention to be actually administered may be appropriately determined by various factors, such as a type of symptoms to be treated, a route of administration, sex, health status, dietary, age and weight of a subject, and the severity of disease.

As used herein, the term "pharmaceutically effective amount" refers to an amount enough to inhibit or alleviate a disease at a reasonable ratio applicable to the medical use. The effective dose level may be determined according to factors including the kind, disease severity, age, and sex of a subject, the activity of a drug, the sensitivity to a drug, the time of administration, the route of administration, the rate of excretion, the duration of treatment, and a medicine to be simultaneously used, and other factors well-known in the medical field. For example, the administration may be performed at a dose of 0.01 to 500 mg/kg per day, specifically, 10 to 100 mg/kg per day, and the administration may be performed once a day or divided into several times.

The composition of the present invention may be administered as an individual therapeutic agent or in combination with other therapeutic agents, and may be sequentially or simultaneously administered together with a conventional therapeutic agent. In addition, the composition may be administered once or multiple times. It is important to administer an amount capable of obtaining a maximum effect with a minimal amount without side effects in consideration of all of the factors, and the amount may be easily determined by those skilled in the art.

The composition of the present invention may be used alone or may be used in combination with surgical operation, hormonal therapy, chemotherapy, and methods using biological response regulators to prevent or treat a vascular smooth muscle cell proliferative disease.

In accordance with still another aspect of the present invention, there is provided a method for screening a target gene of a miRNA that inhibits the proliferation or migration of vascular smooth muscle cells, the method including profiling differentially expressed genes (DEGs) at days 3 and 5 post-injury in the injured arteries.

The "injured arteries", "vascular smooth muscle cells", "proliferation", and "miRNA" are as described above.

The screening method includes: 1) profiling differentially expressed genes (DEGs) at days 3 and/or 5 post-injury in the injured artery; 2) performing in silico target profiling for miR-132-3p, miR-370-3p, miR-130b-5p, and miR-410-3p through target prediction databases by using DEGs; 3) selecting, as target genes for each miRNA, DEGs that are commonly predicted in both rat and human from two or more databases; and 4) identifying whether the selected target genes are down-regulated in the injured arteries.

The profiling in step 1) may indicate a gene expression profiling, and the expression levels of multiple genes may be compared simultaneously between two or more samples. In analyzing gene expression, northern blotting, microarray analysis, RNA sequencing, or the like may be used. In the present invention, the changes in overall gene expression were investigated through RNA sequencing, but the analyzing of gene expression is not limited thereto.

The target prediction databases in step 2) are for predicting target genes of the miRNAs. The functions of miRNAs may usually be understood through the genes regulated by the miRNAs, and as of October 2018, 38,589 human miRNAs (mature miRNAs) have been databased (miRBase database, version 22.1).

The prediction may be made by alignment of seed sequences of 3' UTR and a miRNA, and the precision of prediction is improved by adding structural features or evolutionarily conserved binding sites. Examples of programs providing those prediction results may include DIANA-microT, MicroInspector, MiRanda, PicTar, RNA22, RNAhybrid, TargetBoost, TargetScan, miRDB, and miRmap. In the present invention, TargetScan, miRDB, and miRmap were used, but are not limited thereto.

The target gene in step 3) may specifically refers to a gene, of which the miRNA can regulate intracellular expression by mixing with miRNA through complementary binding to mRNA. There may be numerous target genes for one miRNA.

The screening in step 4) may refer to gene screening and may indicate selecting target genes from an existing library. Examples of the screening method may include screening using hybridization, screening using antibodies, screening using differences in gene expression, screening using binding to specific proteins, and the like. In the present invention, screening using differences in gene expression was used, but is not limited thereto.

### [Mode for Carrying Out the Invention]

Hereinafter, the present invention will be described in detail through examples. These examples are given for specifically illustrating the present invention, and the scope of the present invention is not limited thereto.

### Experimental Example 1. Creation of balloon-induced carotid injury model

A balloon-induced carotid injury model was created by using ten-week-old male albino rats (Sprague-Dawley rats) acclimated to the environment for one week after basic breeding.

Specifically, balloon injury was created using a 2F Fogarty balloon embolectomy catheter in the left common carotid artery of each rat. The left external carotid artery was exposed and arterial branches were electro-coagulated, and then a catheter was inserted through the transverse arteriotomy of the external carotid artery and positioned at a 1 cm distance from a cut-down site. The catheter was then inflated and moved back and forth along the common carotid artery to injure the carotid artery. For catheter-mediated intramural delivery of miRNA inhibitors, the transfection complex (200 nM miRNA/10 µl Lipofectamin RNAimax in 200 µl Opti-MEM) was infused into the injured carotid lumen and then incubated for 15 min. The lumen was washed once with saline before the catheter was removed. After the removal of the catheter, the punched area was sealed and the clamp in the common carotid artery was released to reestablish blood flow. Unless otherwise stated, the rats were then recovered in the cages for up to 14 days.

### Experimental Example 2. Histopathological analysis

The balloon-injured rats were euthanized and the common carotid artery was excised after transcardiac perfusion-fixation with heparinized saline containing 3.7% formaldehyde. The vessels were paraffin embedded and sectioned by rotary microtome (Leica RM2255). The two serial tissue sections (4 µm in thickness) were obtained from the middle area of the common carotid artery and stained with haematoxylin and eosin. The luminal, internal elastic laminal, and external elastic laminal areas were measured using NIH Image v 1.62. The intimal and medial areas were determined by subtraction of the luminal area from the internal elastic area and subtraction of the internal elastic area from the external elastic area, respectively. The values from two serial sections per rat were averaged for analysis.

### Experimental Example 3. RNA sequencing

To perform small RNA and mRNA sequencing, total RNA was isolated from the rat carotid artery tissues using QIAzol Lysis Reagent (Qiagen) according to the manufacturer's protocol. For sham control, three carotid tissues were pooled to increase the amount of total RNA. The purity and integrity of the total RNA extract was determined by NanoDrop 8000 spectrophotometer (Thermo Scientific) and Bioanalyzer (Agilent Technologies), respectively, and the samples with a RIN value greater than 8 were used for further sequencing process.

First, for small RNA sequencing, small RNA libraries were prepared using TruSeq Small RNA Prep kit (Illumina). Briefly, 1 µg of total RNA was ligated with 3' and 5' RNA adapters. Reverse transcription PCR was performed using primers that anneal to the 3' and 5' adapters for creating and enriching cDNA constructs. The cDNA library was purified with the Pippin prep electrophoresis platform (Sage Science), and the quality of the library was verified by 2100 Bioanalyzer (Agilent Technologies). Small RNA sequencing was performed using Hiseq 2500 system (Illumina) with a 1 × 51 setup.

Then, for mRNA sequencing, the mRNA sequencing libraries were prepared using TruSeq RNA Prep kit v2 (Illumina). Briefly, the mRNA samples were purified from 1 µg of total RNA using poly-T oligo-attached magnetic beads, and the fragmented mRNAs were primed with random hexamers and were reverse transcribed. The mRNA templates were removed and the cDNA second strands were synthesized. After 3' A-tailing and 5' end repairing, DNA sequencing adaptors were ligated to the cDNA templates. Then, PCR was performed for amplifying the templates. The quality of the library was verified by 2100 Bioanalyzer (Agilent Technologies), and the mRNA sequencing was performed using Hiseq 2500 system (Illumina) with a 2 × 101 setup.

### Experimental Example 4. Cell culture

Primary human aortic smooth muscle cells (HASMCs) were purchased from Lonza and expanded by sub-culturing in the Smooth Muscle Cell Growth Medium (SmGM) containing 5% fetal bovine serum with growth factors and an antibiotic (cat. no. cc-4149, Lonza). Human aortic smooth muscle cells were mainly used for the experiments at passage number 5-7. HEK293T cells were cultured in DMEM containing 10% fetal bovine serum and 1% penicillin/streptomycin, and all cultures were maintained in a humidified incubator containing 5% CO₂ at 37°C.

The mirVana miRNA mimics or inhibitors (Invitrogen, USA) and siRNA (Bioneer, Korea) were transfected using Lipofectamine RNAimax (Invitrogen).

The cells were plated at a density of 1 × 10⁵ cells/well in a 6-well plate. At 24 h later, the transfection complexes containing miRNA mimics (0.1 nM each), miRNA inhibitors (10-150 nM each), or siRNAs (100 nM each) were added to the culture plate and then changed with fresh culture media after 24-h transfection.

### Experimental Example 5: Real-time quantitative PCR

Total RNA was isolated from the cultured human aortic smooth muscle cells and rat carotid artery tissue by using QIAzol Lysis Reagent (Qiagen) according to the manufacturer's protocol. For the miRNA validation, individual cDNAs were synthesized from 40 ng of the isolated total RNA using TaqMan MicroRNA Reverse Transcription Kit and specific RT primers of TaqMan MicroRNA Assays (Applied Biosystems). Expression levels of mature miRNAs were quantified by quantitative real-time PCR using TaqMan Universal Master Mix II and specific FAM-based probes and primers of TaqMan MicroRNA Assays (Applied Biosystems) according to the manufacturer's protocol.

The thermal cycling conditions for miRNAs were: 10 min at 95°C for initial enzyme activation, followed by 40 cycles of 15 s at 95°C (denaturation) and 60 s at 60°C (annealing/extension) for amplification.

U87, snoRNA, and U6 were used as internal controls for rat carotid arteries. U6 was used as an internal control for human aortic smooth muscle cells.

For the mRNA validation, reverse transcription was performed from 1 µg of the isolated total RNA using ImProm-II RT system (Promega). Quantitative real-time PCR was performed using gene-specific primers (all Qiagen) and SYBR Green (Roche).

The thermal cycling conditions for mRNAs were: 15 min at 95°C for initial denaturation, followed by 40 cycles of 15 s at 94°C (denaturation) and 30 s at 55°C (annealing) and 30 s at 72°C (extension) for amplification.

Melting curve analysis was carried out at the end of the PCR. The β-actin or 18S RNA was used as housekeeping gene. The CFX Connect Real-Time PCR Detection System (Bio-Rad) was used to detect levels of mature miRNAs and transcripts. Relative gene expression was determined by ΔΔCt value.

### Experimental Example 6: Immunoblot analysis

Human aortic smooth muscle cells were rinsed twice with ice-cold phosphate-buffered saline (PBS) and quickly frozen on liquid nitrogen. The cells were lysed in lysis buffer containing 20 mM HEPES (pH 7.0), 1% Triton X-100, 150 mM NaCl, 10% glycerol, 1 mM EDTA (pH 8.0), 2 mM EGTA (pH 8.0), 1 mM DTT, 5 mM Na₃VO₄, 5 mM NaF, 1 mM AEBSF, 5 µg/ml aprotinin, and 5 µg/ml leupeptin. After centrifugation at 12,000×g for 10 min, the purified lysates (30 µg each) were separated on denaturing polyacrylamide gels and transferred to nitrocellulose membranes. The membranes were incubated with primary antibodies in a Tris-buffered saline (TBS) solution containing 0.05% Tween-20 and 5% BSA overnight at 4°C. Next, the membranes were incubated with HRP-conjugated secondary antibodies (1 :3000 dilution) in a TBS solution containing 0.05% Tween-20 and 5% skim milk for 1 h. The immune-reactive bands were visualized using WESTSAVE up ECL solution (cat. no. LF-QC0101, Abfrontier, Korea).

### Experimental Example 7: Cell proliferation and cell cycle analysis

For cell proliferation assay, the miRNA inhibitor-transfected human aortic smooth muscle cells (HASMCs) were seeded at a density of 2000 cells/well on a 96-well plate, grown for the indicated times, and then incubated with a WST-1 reagent (10 µl/well) at 37°C for 1 h. The number of viable cells was estimated by measuring the absorbance at 450 nm.

For cell cycle analysis, human aortic smooth muscle cells (1 × 10⁵ cells) were harvested after 48-h transfection. The cells were fixed and permeabilized in 70% ethanol overnight at -20°C. The cells were treated with 100 µg/ml RNase A for 1 h at 37°C and then stained with 10 µg/ml propidium iodide. The DNA content in cells was measured by FACSCalibur system (BD biosciences), and the percentages of G0 / G1 diploid cells were analyzed by Modfit LT software (Verity Software House).

### Experimental Example 8: Immunofluorescence staining

For tissue staining, the paraffin sections of balloon injured carotid arteries were deparaffinized in xylene and rehydrated in ethanol. The rehydrated tissue sections were boiled for 20 min in a citric acid-based antigen unmasking solution (Vector Laboratories) for antigen retrieval. For dual immunofluorescence, the tissue sections were blocked with 5% normal donkey serum in PBS-T (0.3% Triton X-100 in PBS) for 1 h. Then, the samples were incubated with FITC-conjugated anti-vWF antibody (1:50 dilution, Abcam) overnight at 4°C. After washing with PBS-T three times, the samples were incubated with Cy3-conjugated anti-SMA antibody (1:200 dilution, Sigma Aldrich) for 2 h at room temperature in the dark.

For the F-actin staining, human aortic smooth muscle cells were fixed with 3.7% formaldehyde for 15 min and permeabilized with PBS-T for 15 min at room temperature. The cells were labelled with an Alexa Fluor 488-conjugated phalloidin (cat. no A12379, Invitrogen) for 60 min at room temperature.

For TUNEL assay, the fixed cells were incubated with a permeabilizaiton solution (0.1% Triton X-100, 0.1% sodium citrate) for 2 min at 4°C. The cells were incubated with TUNEL reaction mixture for 60 min at 37°C in the In Situ Cell Death Detection Kit (Roche Diagnostics).

Nuclear DNA was labeled with DAPI, and fluorescence images were obtained using LSM880 Airyscan confocal microscope (Carl Zeiss).

### Experimental Example 9: Transwell migration assay

The chemotactic cell migration was measured using 24-well Transwell culture chambers (Costar; 8-mm pore size). The upper chamber was coated with gelatin B (1 mg/ml) and air-dried for 1 h. Human aortic smooth muscle cells were transfected with miRNA inhibitors for 24 h, serum-starved for 18 h, and re-plated on the upper chambers with basal media at a density of 6000 cells/chamber. Complete media were added to the lower chambers, and Transwell chambers were incubated at 37°C for 24 h. The non-migrated cells were removed from the upper side of the membranes, and the cells passed through and attached to the lower side of the membranes were fixed and stained with 0.6% hematoxylin and 0.5% eosin. The number of stained cells from 4 different fields was counted and averaged.

### Experimental Example 10: Monocyte adhesion assay

Human aortic smooth muscle cells were transfected with the miRNA inhibitors for 24 h and re-plated on a 96-well plate (4,000 cells/well). Then, human aortic smooth muscle cells were stimulated with TNF-α (10 ng/ml) for 18 h. Separately, monocytic U937 cells (1X10⁶ cells/well) were stimulated with IFN-γ (50 µg/ml) for 24 h. The activated U937 cells were labeled with 4 µM tetramethylrhodamine ethyl ester, perchlorate (cat. No. T-669, Molecular Probes) for 30 min. The labeled U937 cells (1 × 10⁵ cells/well) were added to the confluent human aortic smooth muscle cells and incubated at 37 °C for 1 h. The unbound U937 cells were gently removed by washing three times with PBS. The adherent U937 cells were detected using ZOE Fluorescent Cell Imager (Bio-Rad) and counted from two randomized fields.

### Experimental Example 11: Luciferase reporter assay

For the target gene prediction of miR-370-3p, the reporter plasmid pMirTarget containing the full-length 3' UTR sequence of human BMP7 gene (hBMP7-3' UTR) was purchased from Origene (cat. no. SC218118). Two consecutive nucleotides in the miR-370-3p target region #1 (nucleotide 229-235) of the hBMP7-3' UTR were mutated and used as a negative control. For the reporter assay, HEK293T cells were plated on a 24-well plate and transfected with the reporter plasmid for 6 h. The cells were then transfected with a miR-370-3p mimic for 48 h and lysed for a protein assay. The same quantity of cell lysates was subjected to a luciferase assay. The luminescence signal was measured in a VICTOR Multilabel Plate Reader (Perkin Elmer).

### Experimental Example 12: In situ hybridization

The miR-370-3p expression in arterial tissues was detected by the miRCURY LNA miRNA in situ hybridization(ISH) Optimization Kits(Qiagen) according to the manufacturer's protocol. The 5' and 3' DIG-modified has-miR-370-3p miRCURY LNA miRNA Detection Probe was used for hybridization. Briefly, 4-µm paraffin sections of the fixed arterial tissues were deparaffinized and rehydrated. Nuclease was inactivated by proteinase K for 10 min at 37°C, and the hybridization was performed by incubating with the miRNA detection probes (40 nM each) for 1 h at a temperature 30°C lower than the RNA *Tm* value. The slides were washed with a serial dilution of saline-sodium citrate (SSC) hybridization buffer at each annealing temperature, and immune-detection was performed by incubating with an alkaline phosphatase-conjugated anti-DIG antibody (cat. no. 11093274910, Roche) for 60 min at room temperature. For color development, the slides were incubated with an NBT/BCIP (cat. no. 11697471001, Roche) substrate solution containing 0.2 nM Levamisol for 2 h at 37°C. In addition, a scrambled probe annealed to the continuous tissue fragments was used as a control, and the cell nuclei were labeled with Nuclear Fast Red (Sigma).

### Experimental Example 13: Statistical Analysis

Unless otherwise specified, data were analyzed by Student's t-test between two groups and one-way ANOVA with Turkey's test for multiple groups. P < 0.05 was considered to be statistically significant.

### Example 1: Identification of miRNAs differentially expressed in injured arteries

Based on the histological analysis of the neointimal thickening over the injury time from the balloon injury model of the rat carotid artery examined in the previous study, two time points, 3- and 5-day post-balloon injury were selected, and the miRNAs that direct the initiation of smooth muscle cell dedifferentiation and proliferation were examined.

Specifically, total RNA samples were prepared, and separated into small RNAs and messenger RNAs and purified by proper purification procedures. The two RNA pools were subjected to library construction and HiSeq-based sequencing, and the small RNA sequencing data were first analyzed by RSEM software.

As a result, as shown in FIG. 1, 62 miRNAs were differentially expressed at two time points compared with the sham control (FIG. 1A). The miRNAs were examined one by one in terms of novelty as well as relevance to the vascular system, and 50 miRNAs were selected for quantitative verification by miRNA-specific real-time PCR. As a result, 12 up-regulated miRNAs and 6 down-regulated miRNAs showed at least 5-fold change in damaged arteries compared with the sham control, and one-fifth of the miRNAs were transiently down-regulated on day 3 after injury, indicating the potential as early-response miRNAs. Some miRNAs that are not identified in the human database or have mismatched seed sequence between rats and humans were filtered out, and among the remaining miRNA candidates, miRNAs, such as miR-221-3p, miR-21-5p, and miR-146a-5p, are known for overexpression of smooth muscle cells {Sun, 2011 #2627; Liu, 2009 #2623; and Ji, 2007 #2625}, supporting the validity of screening strategies using rodent models.

The top 10 miRNAs, including 8 up-regulated miRNAs and 2 down-regulated miRNAs, showed dramatic expression changes of at least 5-fold in injured carotid arteries (FIG. 1B). To check the human relevance, expression levels thereof in the cultured primary human aortic smooth muscle cells harboring a synthetic phenotype were examined, and as a result, the qPCR analysis indicated that six out of eight up-regulated miRNAs were prominently expressed in the human smooth muscle cells (FIG. 1C).

As such, a subset of miRNAs including some known candidates associated with neointimal hyperplasia was discovered through miRNA screening using the rodent models.

### Example 2. Up-regulated miRNAs controlling in vitro smooth muscle cell functions

To determine the roles of the up-regulated miRNAs in the smooth muscle cell biology, three types of cell-based assays were performed in vitro by using human aortic smooth muscle cells transfected with miRNA inhibitors.

The smooth muscle cell proliferation was significantly inhibited by miR-132-3p and miR-370-3p (FIG. 2A). As an inflammatory indication, the adhesion of monocytes to human aortic smooth muscle cells was markedly inhibited by three miRNAs of miR-130b-5p, miR-132-3p, and miR-410-3p. Based on these cell assays, proliferation-related miRNAs, that is, miR-132-3p and miR-370-3p were deeply investigated. Both the two miRNA inhibitors induced cell cycle arrest at G1 phase, thereby suppressing the time-dependent proliferation of human aortic smooth muscle cells (FIGS. 2B and 2C). Consistently, both miRNA inhibitors markedly augmented the levels of two cyclin-dependent kinase inhibitors, p21 and p27 (FIG. 2D). However, such miRNA inhibition did not induce the apoptotic cell death of human aortic smooth muscle cells, indicating that these inhibited the smooth muscle cell proliferation.

In injured arterial tissues, the phenotype transition of smooth muscle cells from the contractile state to the synthetic state by dedifferentiation should precede the proliferation-dependent neointimal hyperplasia, and therefore, it was examined whether miR-132-3p and miR-370-3p were involved in the phenotype change.

For the simplest way to mimic the in-vivo arterial environment, the contractile phenotypes of human aortic smooth muscle cells were induced by culturing at high confluency on a laminin-coated plate. When the highly confluent smooth muscle cells were re-plated at low confluency, the cells became gradually transformed into the synthetic phenotype. Immunoblot analysis in Experimental Example 6 confirmed that the transfection of the miR132-3p inhibitor restored the SMA level, which had disappeared at low confluency (FIG. 2E). Indeed, the immunofluorescence staining of F-actin filament demonstrated that the synthetic phenotype of human aortic smooth muscle cells was transformed into the contractile phenotype by the miR-132-3p inhibitor, at low confluency (FIG. 2F).

Thus, it is likely that miR-132-3p and miR-370-3p each can regulate phenotype transition and proliferation signaling.

### Example 3. Up-regulated miRNAs regulating neointimal hyperplasia in vivo

To evaluate the in vivo miRNA efficacy in the balloon injury model, the arterial expression levels of four functional miRNAs were examined through the in situ hybridization as in Experimental Example 12.

As a result, the stained images showed that the expression of two proliferation-related miRNAs (miR-132-3p and miR-370-3p) was markedly increased in the balloon-injured carotid arteries compared with those in the sham control (FIG. 3A), and the two other inflammation-related miRNAs (miR-130b-5p and miR-410-3p) were also substantially induced by the balloon injury. The in vivo function of the four miRNAs in the balloon-injured rat arteries was evaluated.

Compared with the control, the catheter-mediated local intramural delivery of the miRNA inhibitors markedly reduced the neointimal hyperplasia in the balloon-injured lesion (FIG. 3B). The immunofluorescence staining of carotid arteries against von Willebrand (vWF) and SMA as the EC and smooth muscle cell markers showed that the miR-130b-5p inhibitor facilitated the recovery of the EC monolayer, called re-endothelialization (FIG. 3C), and miR-130b-5p exhibited an anti-proliferation function in EC unlike the smooth muscle cells.

These results confirmed that the four miRNAs play distinct roles, such as vascular inflammation and proliferation, in arterial homeostasis.

### Example 4. Identification of target genes of functional miRNAs

Next, to understand the biological consequences of the miRNAs selected in Example 3, mRNA sequencing was performed and differentially expressed genes (DEGs) in carotid arteries of the balloon-injured rats were subjected to profiling.

As a result, as shown in FIG. 4, 3,699 DEGs were significantly changed at days 3 and 5 post-injury compared with those in the sham control (FIG. 4A). The heatmap analysis showed that the up-regulated DEGs and down-regulated DEGs were approximately half each (FIG. 4B). Using these DEGs, in silico target profiling was performed on the four selected miR-132-3p, miR-370-3p, miR-130b-5p, and miR-410-3p through the target prediction databases (TargetScan, miRDB, and miRmap), and the DEGs that were commonly predicted in both rat and human databases with high prediction scores (TargetScan<-0.1, miRDB>70, miRmap>70) were considered as reliable targets of each miRNA. The gene ontology (GO) enrichment analysis further classified the target gene pools functionally associated with cognate miRNAs, and then among the genes selected through real-time PCR, down-regulated genes in the balloon injured carotid arteries compared with the sham control were identified (FIG. 4C). To show the human relevance, the expression of the target genes in the human aortic smooth muscle cells transfected with miRNA inhibitors was further examined.

Consequently, the real-time PCR revealed a key target gene for each miRNA, among which BMP7, TSPAN2, SOCS2, and SMAD6 are specific targets for miR-370-3p, miR-130b-5p, miR-132-3p, and miR-410-3p, respectively (FIG. 4D).

### Example 5. miR-370/BMP7 axis essential for smooth muscle cell proliferation

Especially, miR-370 was detected in the blood samples of patients with unstable angina, type II diabetes, and hyperlipidemia [Hoekstra, 2010 #2635; Motawae, 2015 #2636; and Gao, 2012 #2637], and thus to examine the biological significance of the miR-370/bone morphogenetic protein (BMP)-7 axis in the proliferation of smooth muscle cells, the detection of miR-370 was attempted in the human cell and arterial tissues.

The in-situ hybridization demonstrated a higher expression of miR-370-3p in the coronary tissue sections from human patients with atherosclerosis type II and IV lesions (FIG. 5A), validating that miR-370 is a novel miRNA associated with atherosclerosis. Thereafter, the relationship between miR-370 and BMP7 was examined in the cultured human aortic smooth muscle cells, and indeed, serum stimulation induced miR-370-3p expression but reduced BMP7 expression (FIG. 5B). Consistently, reporter analysis using the 3'-UTR region of human BMP7 revealed that the miR-370 mimic evidently reduced the expression of luciferase containing wild-type UTR but not mutant UTR (FIG. 5D). In addition, western blot analysis distinctly demonstrated that miR-370 inhibition increased the protein level of BMP7 in human smooth muscle cells (FIG. 5D).

These results collectively confirmed that the BMP7 gene is a true target gene of miR-370 in the human smooth muscle cells. Since the recombinant BMP7 treatment was reported to inhibit the small muscle cell proliferation [Lagna, 2007 #2641; and Dorai, 2000 #2640], the involvement of miR-370/BMP7 axis in the proliferation of smooth muscle cells was investigated. As a direct evidence, the BMP7 treatment markedly induced the SMAD1/5/9 phosphorylation in human aortic smooth muscle cells (FIG. 5E), suggesting an anti-mitogenic effect of BMP7 in smooth muscle cells. Additionally, the cell proliferation analysis demonstrated that BMP7 depletion restored the proliferation of smooth muscle cells, which were inhibited by the miR-370 inhibitor (FIG. 5F). Therefore, it was confirmed that miR-370-dependent control of BMP7 expression is a prerequisite for smooth muscle cell growth and neointimal hyperplasia occurring in the injured arteries.

Taken together, the miRNA inhibitors of the present invention, specifically, each of miR-132-3p, miR-370-3p, miR-130b-5p, and miR-410-3p inhibitors has a proliferation inhibitory effect on smooth muscle cells, and thus it can be expected that the inclusion of the miRNA inhibitors in smooth muscle cells is effective in the prevention or treatment of a vascular smooth muscle cell proliferative disease.

While the present invention has been described with reference to the particular illustrative embodiments, a person skilled in the art to which the present invention pertains can understand that the present invention may be embodied in other specific forms without departing from the technical spirit or essential characteristics thereof. In this regard, the examples described above should be understood to be illustrative rather than restrictive in every respect. The scope of the invention should be construed that the meaning and scope of the appended claims rather than the detailed description and all changes or variations derived from the equivalent concepts fall within the scope of the present invention.

## Claims

1. A pharmaceutical composition for prevention or treatment of a vascular smooth muscle cell proliferative disease, the pharmaceutical composition comprising a microRNA (miRNA) inhibitor as an active ingredient.

2. The pharmaceutical composition of claim 1, wherein the miRNA is at least one selected from the group consisting of miR-132-3p, miR-370-3p, miR-130b-5p, and miR-410-3p.

3. The pharmaceutical composition of claim 1, wherein the miRNA inhibitor regulates in vitro vascular smooth muscle cell functions and in vivo neointimal hyperplasia by inhibiting an up-regulated expressed miRNA in the injury artery.

4. The pharmaceutical composition of claim 1, wherein the miRNA inhibitor is any one selected from the group consisting of an siRNA, an aptamer, an antisense oligonucleotide, a ribozyme, and a compound, specific to a miRNA.

5. The pharmaceutical composition of claim 1, wherein the vascular smooth muscle cell proliferative disease is selected from the group consisting of vascular stenosis, vascular restenosis, atherosclerosis, atherosclerotic arteriosclerosis, heart failure, myocardial infarction, angina, arrhythmia, hypertensive heart disease, congenital heart disease, stroke, and peripheral vascular stenosis.

6. A kit for diagnosis of a vascular smooth muscle cell proliferative disease, the kit comprising an agent capable of detecting a miRNA.

7. The kit of claim 6, wherein the miRNA is selected from the group consisting of miR-132-3p, miR-370-3p, miR-130b-5p, and miR-410-3p.

8. The kit of claim 6, wherein the kit is selected from the group consisting of a microarray, an aptamer chip kit, an enzyme linked immunosorbent assay (ELISA) kit, a serial analysis of gene expression (SAGE) kit, a quantitative real time PCR (qRT-PCR) kit, and a combination thereof.

9. An information-providing method for diagnosing a vascular smooth muscle cell proliferative disease, the method comprising:
measuring the expression level of a miRNA in an isolated biological sample; and
determining that there is a risk of developing a vascular smooth muscle cell proliferative disease when the expression level of the miRNA is increased compared with that in a control group.

10. The information-providing method of claim 9, wherein the miRNA is selected from the group consisting of miR-132-3p, miR-370-3p, miR-130b-5p, and miR-410-3p.

11. The information-providing method of claim 9, further comprising determining whether the expression level of a miRNA target gene in the biological sample is reduced compared with the control group.

12. The information-providing method of claim 8, wherein the isolated biological sample is a blood or a coronary tissue section from a patient with atherosclerosis.

13. A method for treating a vascular smooth muscle cell proliferative disease, the method comprising administering, to a non-human subject in need thereof, a pharmaceutical composition for prevention or treatment of a vascular smooth muscle cell proliferative disease, the pharmaceutical composition containing a miRNA expression inhibitor and a pharmaceutically acceptable carrier.

14. The method of claim 13, wherein the miRNA is selected from the group consisting of miR-132-3p, miR-370-3p, miR-130b-5p, and miR-410-3p.
